# EUROPÄISCHE PATENTSCHRIFT

(11) **EP 2 158 136 B1**
(45) Veröffentlichungstag und Bekanntmachung des Hinweises auf die Patenterteilung: **08.08.2012**
(21) Anmeldenummer: 08784227.4
(22) Anmeldetag: 19.06.2008
(51) Int. Cl.: B65D 47/20

(54) **VORRICHTUNG ZUM AUSTRAGEN EINER FLIESSFÄHIGEN SUBSTANZ**
APPARATUS FOR DISCHARGING A POURABLE SUBSTANCE
DISPOSITIF DE DÉVERSEMENT D'UNE SUBSTANCE APTE À L'ÉCOULEMENT

(30) Priorität: 20.06.2007 DE 202007008646 U
(43) Veröffentlichungstag der Anmeldung: 03.03.2010
(73) Patentinhaber: Sulzer Mixpac AG, 9469 Haag (CH)
(72) Erfinder: SOGARO, Alberto, C., 61476 Kronberg (DE)
(74) Vertreter: Grünberg, Thomas
(86) Internationale Anmeldenummer: PCT/DE2008/001022
(87) Internationale Veröffentlichungsnummer: WO 2008/154912

(56) Entgegenhaltungen:
- EP-A- 0 111 796
- EP-A- 1 782 856
- DE-U1-202006 002 926

## Beschreibung

Die Erfindung betrifft eine Vorrichtung zum Austragen einer fließfähigen Substanz, umfassend eine Außenhülse, die als Behälter für die fließfähige Substanz ausgebildet, und mindestens einen Einsatz, der an einer Stirnseite geöffnet ist und an der anderen Stirnseite geschlossen ist und an seinem Umfang mindestens eine Öffnung hat, wobei die Außenhülse gegenüber dem Einsatz zwischen einer Sperrstellung, in der ein Fluidstrom zwischen der Außenhülse und dem Einsatz mittels einer Dichteinrichtung gesperrt ist, und einer Aktivierungsstelle verlagerbar ist, in welcher der Fluidstrom zwischen der Außenhülse und dem Einsatz freigegeben ist.

Eine derartig ausgebildete Austragvorrichtung ist aus der EP 1 782 856 A1 bekannt. Diese Austragvorrichtung umfasst einen komprimierbaren Vorratsabschnitt und einen Einsatz aus einem Hartkunststoff, der eine Applizierspitze aufweist und im offenen Ende des komprimierbaren Abschnitts verschiebbar geführt ist. Der Einsatz umfasst eine Querbohrung, die mit einer zu der Applizierspitze führenden Axialbohrung verbunden ist. Die Querbohrung bzw. deren Öffnungen wirken mit einer an der Außenhülse ausgebildeten Dichtlippe zusammen, so dass in einer Sperrstellung ein Fluidstrom zwischen dem von der Außenhülse gebildeten Vorratsraum und der Querbohrung gesperrt und in einer Aktivierungsstellung dieser Fluidstrom freigegeben ist. Zur Aktivierung wird mithin der Einsatz gegenüber der Außenhülse so verschoben, dass die Querbohrung bzw. deren Öffnungen über die Dichtlippe gefahren werden. Der Versatz zwischen dem Einsatz und der Außenhülse bzw. dem Vorratsbehälter erfolgt durch einander entgegen gerichteten axialen Druck auf diese beiden Bauteile. Ein solcher axialer Druck ist schwer zu dosieren.

Der Erfindung liegt die Aufgabe zugrunde, eine Vorrichtung der vorstehend beschriebenen Art zu schaffen, bei dem in einfacher Weise ein definierter Versatz zwischen dem zylindrischen Einsatz und der Außenhülse realisierbar ist.

Diese Aufgabe ist erfindungsgemäß durch die Vorrichtung mit den Merkmalen des Anspruchs 1 gelöst.

Die Vorrichtung nach der Erfindung weist mithin ein Ventil auf, das einen bereichsweise zylindrischen, insbesondere eine Innenhülse bildenden Einsatz, der an einer Stirnseite offen ist und an der anderen Stirnseite geschlossen ist und an seinem Umfang mindestens eine Öffnung hat, die mit der offenen Stirnseite verbunden ist, sowie eine Außenhülse umfasst, die gegenüber dem Einsatz zwischen einer Sperrstellung, in der ein Fluidstrom zwischen der Außenhülse und der Öffnung des Einsatzes mittels einer Dichteinrichtung gesperrt ist, und einer Aktivierungsstellung verlagerbar ist, in welcher der Fluidstrom zwischen der Außenhülse und der Öffnung des Einsatzes freigegeben ist. Um auf einfache Weise die Aktivierungsstellung augehend von der Sperrstellung realisieren zu können bzw. die Sperrstellung ausgehend von der Aktivierungsstellung realisieren zu können, hat der Einsatz erfindungsgemäß ein Außengewinde, das in die Außenhülse eingreift, sowie einen Ringbund, der ein Anschlag für den Einsatz an der Außenhülse und ein Betätigungsmittel zum Verlagern der Außenhülse gegenüber dem Einsatz ist.

Der Kern der Erfindung besteht mithin darin, dass durch ein Verdrehen des Einsatzes gegenüber der Außenhülse die Sperrstellung bzw. die Aktivierungsstellung eingestellt werden kann. Solche Drehbewegungen können durch manuelles Greifen der Innenhülse an dem Ringbund sehr präzise und winkelgenau durchgeführt werden, ohne die Komponenten des Ventils zu beschädigen.

Um einem Benutzer der Vorrichtung nach der Erfindung einen haptischen Eindruck beim Erreichen der Aktivierungsstellung zu vermitteln, bildet der Ringbund einen Anschlag zwischen dem Einsatz und der Außenhülse, welcher die Aktivierungsstellung definiert. Denkbar ist ferner auch mindestens eine Raststellung zwischen dem Einsatz und der Außenhülse.

Bei einer bevorzugten Ausführungsform der Vorrichtung nach der Erfindung weist die Außenhülse ein Innengewinde auf, in das das Außengewinde des Einsatzes eingreift. Das Gewinde ist stets stromab der Öffnung des Einsatzes angeordnet. Ausgehend von der Sperrstellung überfährt die Öffnung des Einsatzes beim Einschrauben desselben in die Außenhülse die Dichteinrichtung, so dass der Fluidstrom zwischen dem Innenraum der Außenhülse und der Öffnung des Einsatzes und zu dessen stirnseitiger Öffnung freigegeben ist.

Bei einer alternativen Ausführungsform der Vorrichtung nach der Erfindung ist das Außengewinde des Einsatzes ein selbstschneidendes Gewinde. Dieses schneidet sich mithin das ein Gegengewinde bildende Innengewinde der Außenhülse beim Herstellen der Aktivierungsstellung selbst.

Bei einer speziellen Ausführungsform der Vorrichtung nach der Erfindung umfasst die Dichteinrichtung zwei ringförmige Dichtlippen, die an der Innenseite der Außenhülse ausgebildet sind und deren Abstand größer ist als die maximalen Abmessungen der Öffnung in axialer Richtung des Einsatzes. Damit ist in Sperrstellung des Ventils in axialer Richtung beidseits eine Dichtigkeit des Ventils gewährleistet.

Der Einsatz der Vorrichtung nach der Erfindung ist in seiner einfachsten Form hülsenartig bzw. topfartig ausgebildet. Die Umfangswand des Einsatzes ist dann mit der mindestens einen Öffnung bzw. dem mindestens einen Durchbruch versehen.

Die Außenhülse bildet einen Behälter für einen fließfähigen Stoff, der nach einem Verdrehen des Einsatzes gegenüber der Außenhülse und Freigabe der Öffnung in den Einsatz strömen kann. Der Behälter kann in vielfältiger Weise ausgebildet sein und beispielsweise einen blasenartigen, einen röhrchenartigen, einen tubenartigen, einen kegelartigen oder auch einen wulstartigen Abschnitt aufweisen, in dem der fließfähige Stoff vorgehalten wird.

Des Weiteren kann die Vorrichtung nach der Erfindung mit einem automatischen Rückstellmechanismus versehen sein, so dass sich das Ventil nach dem Öffnen und nach dem Lösen einer Öffnungskraft selbsttätig in seine Sperrstellung versetzt.

Die Vorrichtung nach der Erfindung eignet sich insbesondere zum Applizieren pharmazeutischer oder kosmetischer Stoffe am menschlichen oder tierischen Körper. Der Einsatz kann dann entsprechen dem jeweiligen Anwendungsfall ausgebildet sein. Beispielsweise kann der Einsatz eine Pipettierspitze darstellen oder auch einen Pinsel oder ein Schwämmchen umfassen. Eine pharmazeutische Substanz, die mittels dieser Vorrichtung applizierbar ist, ist beispielsweise ein Gewebekleber, ein Dentalkleber oder dergleichen.

Die Applizierspitze des Einsatzes kann eine Pipetierspitze sein. Zum Applizieren einer in der Außenhülse vorgehaltenen Substanz wird der Einsatz gegenüber der Außenhülse in die Aktivierungsstellung verdreht, so dass die Substanz aus der Außenhülse über die Öffnungen bzw. einen Querkanal und einen sich an diesen anschließenden Axialkanal mittels. der Applizierspitze aufgetragen werden kann. In diesem Falle kann die Außenhülse von einem Pipettierröhrchen gebildet sein, das zumindest bereichsweise aus einem elastisch verformbaren Kunststoff besteht, so dass durch einen radialen Druck auf die Außenhülse in Aktivierungsstellung die fließfähige Substanz zu dem Einsatz bzw. der Pipettierspitze gefördert werden kann.

Der zylindrische Einsatz ist vorzugsweise aus einem Hartkunststoff gefertigt und stellt insbesondere ein Kunststoffspritzgießteil dar. Denkbar ist es aber auch, dass der Einsatz aus einem elastisch verformbaren Kunststoff gebildet ist.

Auch kann der Einsatz mit einem Aufnahmeraum versehen sein, aus dem durch radialen manuellen Druck auf den Einsatz ein in diesem vorgehaltener Stoff über einen Applizierbereich ausgebracht werden kann. Eine derart ausgelegte Vorrichtung eignet sich auch für ein Zwei- oder Mehrkomponentensystem, wobei eine Komponente in der Außenhülse bzw. deren Vorratsraum und eine zweite Komponente in dem Aufnahmeraum des Einsatzes vorgehalten werden kann. Nach dem Aktivieren der Vorrichtung durch Öffnen des erfindungsgemäß ausgelegten Ventils können die Komponenten zur anschließenden Applikation in dem Aufnahmeraum des Einsatzes oder auch in dem Vorratsraum der Außenhülse vermengt werden.

Die erfindungsgemäß ausgebildete Vorrichtung zum Austragen einer fließfähigen Substanz ist insbesondere als Einwegvorrichtung ausgelegt, bei der die Außenhülse vorab befüllt ist. Im deaktivierten, eine Sperrstellung darstellenden Zustand ist die fließfähige Substanz durch die Dichteinrichtung gesichert in der Außenhülse gehalten, die dann eine Vorratseinrichtung darstellt. Dieser Zustand stellt in der Regel den Lieferzustand der Vorrichtung dar. Der Anwender muss zur Aktivierung der Vorrichtung nur die Außenhülse und den Einsatz gegeneinander verdrehen, so dass diese teleskopiert werden und ein Übergang von der geschlossenen Speicherposition in die offene Freigabeposition der Vorrichtung erfolgt. Beim Aktivieren überstreicht die Dichteinrichtung die Öffnung des Einsatzes, so dass die fließfähige Substanz aus der Außenhülse in die Öffnung des Einsatzes fließen kann.

Denkbar ist es auch, eine erfindungsgemäß ausgebildete Vorrichtung zum Austragen einer fließfähigen Substanz mehrstufig auszubilden. In diesem Falle kann die Außenhülse eine Vielzahl von in axialer Richtung hintereinander angeordneten Dichtlippen aufweisen, die jeweils entsprechend dem Durchmesser der Öffnung ausgebildet sind. Der Einsatz kann dann nach Art eines Verdrängungskolbens wirken, der sukzessive in die Außenhülse eingeschraubt wird. Beim Überstreichen jeder Dichtlippe wird eine bestimmte Substanzmenge aus der Außenhülse durch die mindestens eine Öffnung des Einsatzes zu dessen offener Stirnseite zur Applikation gefördert. Insbesondere kann auf diese Weise eine so genannte Kalenderpackung realisiert werden, die mit einer entsprechenden Skalierung versehen sein kann, welche einem Benutzer den exakten Verstellbetrag zwischen dem Einsatz und der Außenhülse vorgibt.

Ferner kann die Vorrichtung nach der Erfindung auch mit einer Aktivierungssicherung bzw. Transportsicherung beispielsweise in Form einer Reißlasche oder dergleichen versehen sein, die zwischen dem Einsatz und der Außenhülse angeordnet ist.

Des Weiteren kann die den Behälter bildende Außenhülse auch zwei Kammern umfassen, die in dessen axialen Richtung hintereinander angeordnet sind und durch eine Trennwand voneinander getrennt sind, wobei beide Kammern jeweils mit einem von zwei bereichsweise zylindrischen Einsätzen der vorstehend beschriebenen Art zusammenwirken, die jeweils einer Applikationsspitze zugeordnet sind. Die Einsätze sind jeweils über ein Gewinde in einem entsprechenden Führungsabschnitt der Außenhülse geführt und damit jeweils zwischen einer Aktivierungsstellung und einer Sperrstellung verstellbar.

Weitere Vorteile und vorteilhafte Ausgestaltungen des Gegenstandes der Erfindung sind der Beschreibung, der Zeichnung und den Ansprüchen entnehmbar.

Zwei Ausführungsbeispiele einer erfindungsgemäß ausgebildeten Vorrichtung sind in der Zeichnung schematisch vereinfacht dargestellt und werden in der nachfolgenden Beschreibung näher erläutert. Es zeigt
- Fig. 1: eine schematische Darstellung einer Pipettiervorrichtung nach der Erfindung;
- Fig. 2: einen Längsschnitt durch die Pipetiervorrichtung;
- Fig. 3: eine Außenhülse der Pipetiervorrichtung in Alleinstellung;
- Fig. 4: die Außenhülse in einer Schnittdarstellung;
- Fig. 5: eine perspektivische Darstellung eines Einsatzes der Pipettiervorrichtung, der in die Außenhülse eingeschraubt ist;
- Fig. 6: einen Längsschnitt durch den Einsatz; und
- Fig. 7: einen Längsschnitt durch eine zweite Ausführungsform einer Vorrichtung nach der Erfindung im zerlegten Zustand.

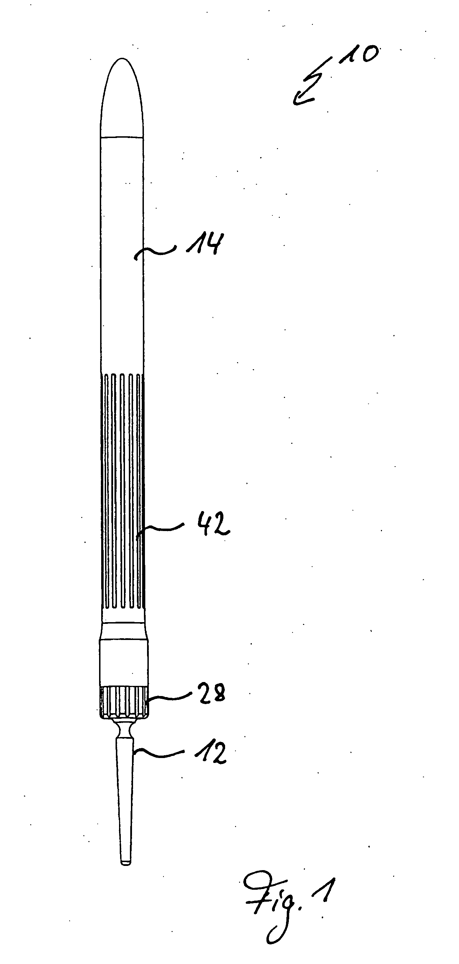

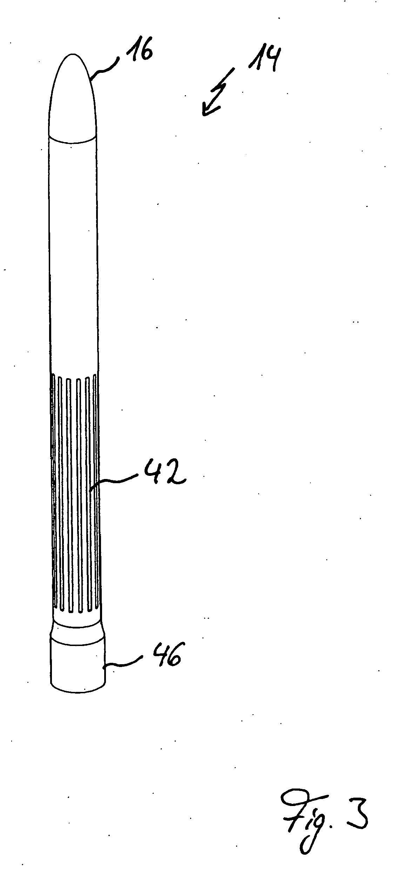

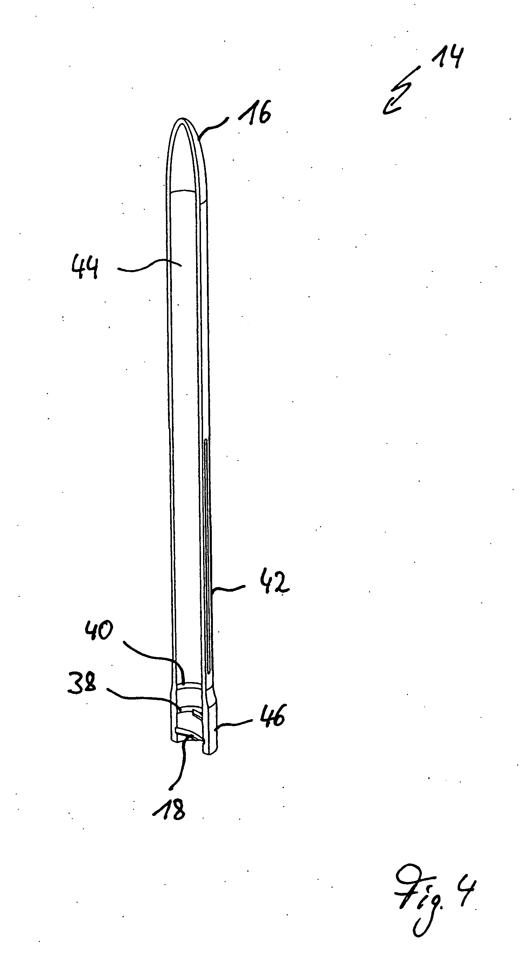

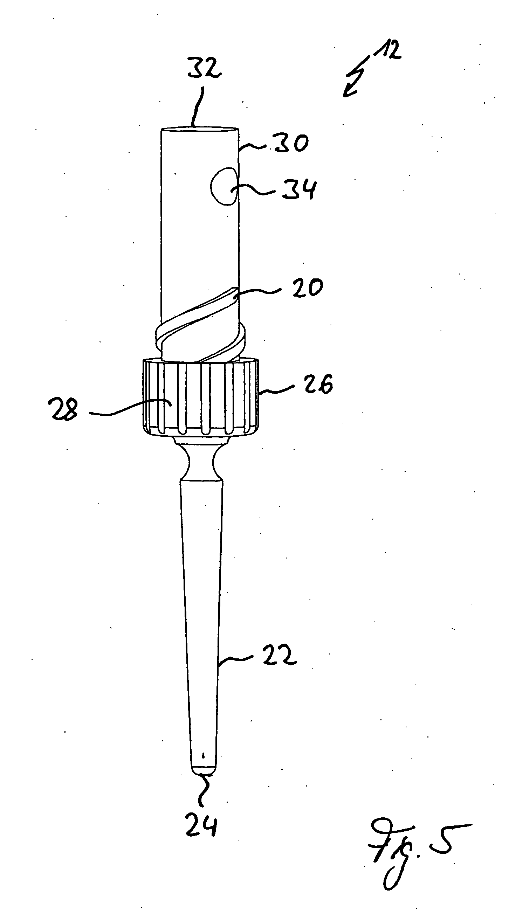

In den Figuren 1 bis 6 ist eine Appliziervorrichtung 10 dargestellt, die beispielsweise zum Pipetieren bzw. Auftragen einer fließfähigen Substanz dient und die aus einem Einsatz 12 und einer Außenhülse 14 gebildet ist, die einen Vorratsbehälter 10 für die fließfähige Substanz darstellt.

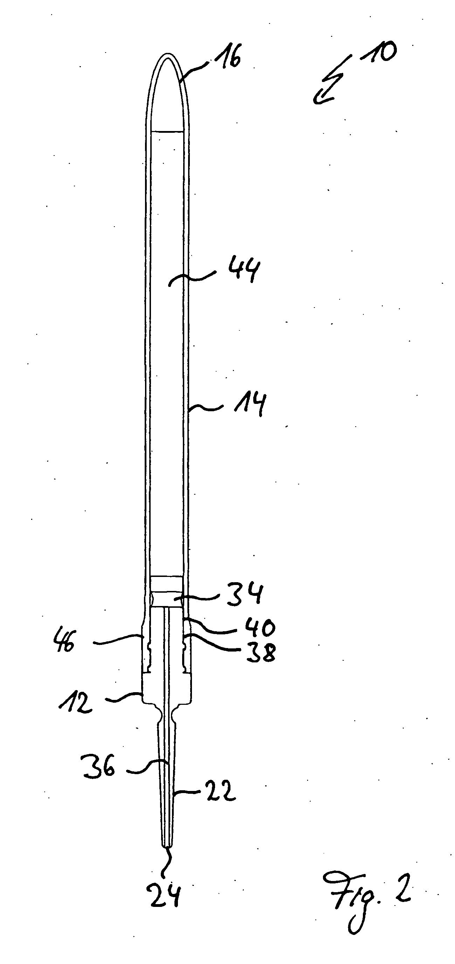

Die Außenhülse 14, die in Fig. 2 in Alleinstellung dargestellt ist, ist röhrchenartig ausgebildet und an einem Ende mit einer geschlossenen Stirnseite 16 versehen. An der zweiten Stirnseite ist die Außenhülse 14 offen ausgebildet, so dass der Einsatz 12 eingreifen kann.

In einem sich an das offene Ende anschließenden Bereich ist die Außenhülse 14 an ihrer Innenseite mit einem Innengewinde 18 versehen, das mit einem Außengewinde 20 des Einsatzes 12 korrespondiert.

Der Einsatz 12 weist eine als Pipettierspitze ausgebildete Appliziereinrichtung 22 auf, die stirnseitig mit einer Applizieröffnung versehen ist und an die sich Pinselhaar, eine Beflockung oder dergleichen anschließen kann, worüber die Substanz auf eine Applikationsfläche aufgetragen werden kann. An der der Applizieröffnung 24 abgewandten Seite schließt sich an die Pipettierspitze 22 ein Ringbund 26 an, der an seiner Umfangsfläche mit einer sich in axialer Richtung des Einsatzes 12 erstreckenden Riffelung 28 versehen ist, die als Greifhilfe dient. Der Ringbund 26 stellt mithin ein Betätigungsmittel der Appliziervorrichtung 10 dar.

An den Ringbund 26 schließt sich wiederum ein zylindrischer bzw. zapfenartiger Abschnitt 30 an, der eine geschlossene Stirnseite 32 aufweist und mit einem Queröffnungen bildenden Querkanal 34 versehen ist. Der Querkanal 34 ist mit einem Axialkanal 36 verbunden, der zu der Applizieröffnung 24 der Pipettierspitze 22 führt.

Des Weiteren ist an dem zylindrischen Abschnitt 30 im Anschluss an den Ringbund 26 das Außengewinde 20 ausgebildet, das in das Innengewinde der Außenhülse 14 eingreift.

Die Außenhülse 14 weist an ihrer Innenseite im Anschluss an das Innengewinde 18 zwei radial nach innen vorspringende Dichtlippen 38 und 40 auf, deren Abstand etwas größer ist als der Durchmesser des Querkanals 34. An ihrer Außenseite ist die Außenhülse 14 mit einer eine Greifhilfe darstellenden Riffelung 42 versehen.

Die Außenhülse 14 ist ein Zweikomponenten-Spritzgießteil. Im Bereich der Riffelung 42 ist sie aus einem elastisch verformbaren Weichkunststoff und in einem Eingriffsbereich 46 für den Einsatz 12, d. h. dem Bereich, in dem der Einsatz 12 über das Außengewinde 20 an dem Innengewinde 18 der Außenhülse 14 und den Dichtlippen 38 und 40 geführt ist, ist sie aus einem Hartkunststoff gebildet. Stromauf des aus dem elastisch verformbaren Weichkunststoff gebildeten Bereichs, d. h. in dem sich der Riffelung 42 an der dem Einsatz 12 abgewandeten Seite anschließenden Bereich, ist die Außenhülse 14 ebenfalls aus einem Hartkunststoff gebildet.

Der Einsatz 12 ist ein Spritzgießteil aus einem Hartkunststoff und kann im Bereich der Pipetierspitze 22 plastisch verformbar ausgebildet sein. Des Weiteren kann im Bereich der Pipetierspitze 22 eine Beflockung zum Applizieren des in der Außenhülse 14 vorgehaltenen Stoffes aufgebracht sein.

Die Anwendung der in den Figuren 1 bis 6 dargestellten Appliziervorrichtung 10 erfolgt in nachfolgend beschriebener Weise.

Im Lieferzustand befindet sich der Einsatz 12 und die Außenhülse 14 in einer Sperrstellung, d. h. in einer Relativstellung, in der das Außengewinde 20 des Einsatzes 12 zwar in das Innengewinde 18 der Außenhülse 14 eingreift, der Querkanal 34 aber zwischen den beiden Dichtlippen 38 und 40 an der Innenseite der Außenhülse 14 angeordnet ist. Damit ist ein Strom einer in der Außenhülse 14 vorgehaltenen Substanz zu dem Querkanal 34 und von dort über den Axialkanal 36 zu der Applizieröffnung 24 gesperrt.

Zur Aktivierung werden nun der Einsatz 12 und die Außenhülse 14 so gegeneinander verdreht, dass die Öffnungen des Querkanals 34 die Dichtlippe 40 überstreichen und der Ringbund 26 mit seinem dem zylindrischen Abschnitt 30 zugewandten Bereich an der Stirnfläche der Außenhülse 14 anschlägt. Der Ringbund 26 bildet mithin auch einen Anschlag für den Einsatz 12. Damit ist die Aktivierungsstellung hergestellt. Nun kann durch radialen manuellen Druck auf den im Bereich der Riffelung 42 angeordneten, aus einem elastisch verformbaren Kunststoff hergestellten Abschnitt der Außenhülse 14 der in einem Vorratsraum 44 der Außenhülse 14 vorgehaltene Stoff über den Querkanal 34 und den Axialkanal 36 zu der Applizieröffnung 24 gefördert und auf eine Applizierfläche beispielsweise eines menschlichen Körpers aufgetragen werden.

Durch Verdrehen des Einsatzes 12 gegenüber der Außenhülse 14 in der Gegenrichtung, wodurch der Querkanal 34 wieder in den zwischen den Dichtlippen 38 und 40 liegenden Bereich verlagert wird, kann die Appliziervorrichtung 10 in einfacher Weise wieder in ihre Sperrstellung versetzt werden. Es handelt sich bei der Appliziervorrichtung 10 mithin um eine wiederverschließbare Vorrichtung.

Die Verbindung des Einsatzes 12 mit der Außenhülse 14 stellt auch eine Sicherung gegen ungewolltes Herausziehen des Einsatzes 12 aus der Außenhülse 14 dar.

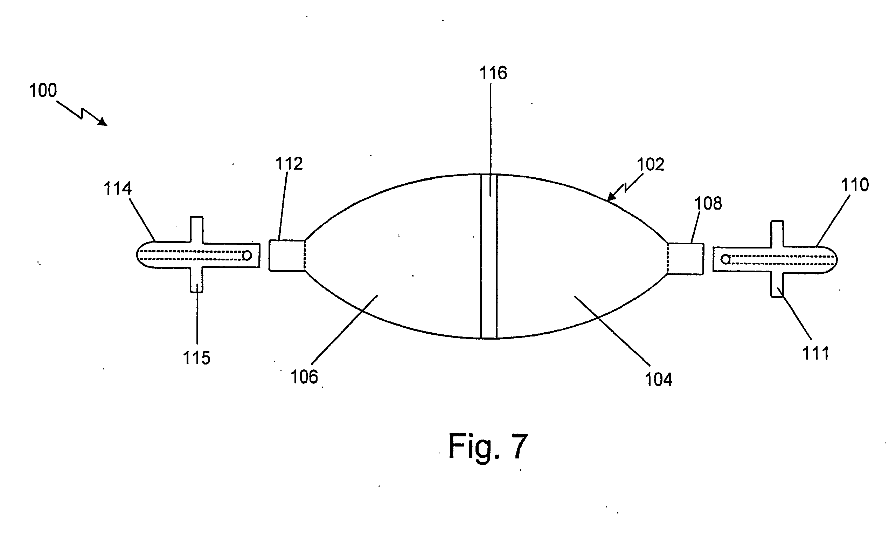

In Fig. 7 ist eine alternative Ausführungsform einer Vorrichtung 100 nach der Erfindung dargestellt. Diese Vorrichtung unterscheidet sich von derjenigen nach den Figuren 1 bis 6 dadurch, dass sie zum Applizieren von zwei Komponenten ausgelegt ist und zu diesen Zwecken mit einem Pipetierröhrchen 102 versehen ist, das zwei Kammern 104 und 106 aufweist, die jeweils zur Aufnahme einer der Komponenten dient.

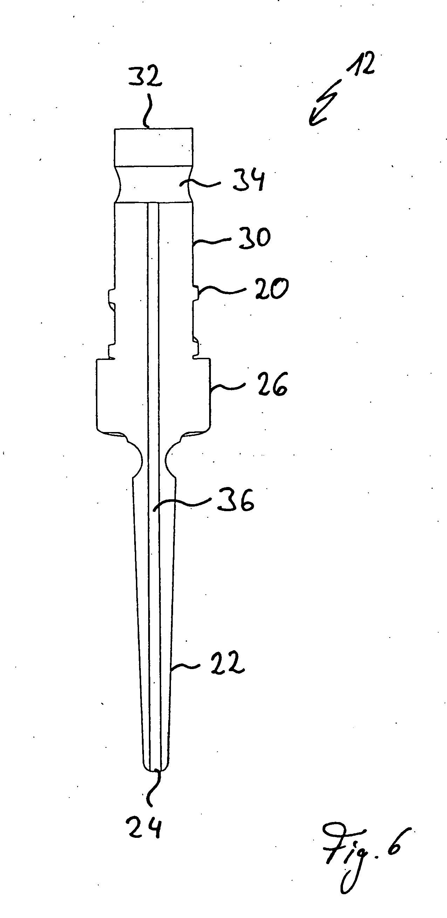

Das Pipetierröhrchen 102 ist entsprechend dem Pipetierröhrchen gemäß der Ausführungsform nach den Figuren 1 bis 6 als Zweikomponenten-Spritzgießteil ausgebildet. Es weist einen ersten Hartbereich 108 zur Führung eines ersten Einsatzes 110 und einen zweiten Hartbereich 112 zur Führung eines zweiten Einsatzes 114 auf. Die Einsätze 110 und 114 sind entsprechend dem Einsatz 12 der Ausführungsform nach den Figuren 1 und 6 ausgebildet und sind mithin jeweils mit einer Applizierspitze versehen. Die Hartbereiche 108 und 112, die jeweils aus einem Hartkunststoff gebildet sind, sind jeweils entsprechend dem Eingriffsbereich 46 der Ausführungsform nach den Figuren 1 bis 6 ausgebildet und bilden mithin jeweils einen als Außenhülsenabschnitt ausgebildeten Führungsabschnitt für die Einsätze 110 bzw. 114. Die Einsätze 110 und 114 sind mithin jeweils zum Verstellen zwischen einer Sperrstellung und einer Aktivierungsstellung mittels eines Gewindes gegenüber dem Pipetierröhrchen 102 verdrehbar, wobei ein jeweiliger Ringbund 111 bzw. 115 als Anschlag und Betätigungsmittel dient.

Im Bereich der Kammern 104 und 106 ist das Pipetierröhrchen 102 jeweils aus einem elastisch verformbaren Weichkunststoff hergestellt, so dass die in der jeweiligen Kammer 104 bzw. 106 vorgehaltene Substanz durch radialen manuellen Druck in der Aktivierungsstellung des jeweiligen Einsatzes 110 bzw. 114 über den betreffenden Querkanal und den betreffenden Axialkanal zu der Applizieröffnung des betreffenden Einsatzes gefördert und auf eine Applizierfläche aufgetragen werden kann.

Zwischen den Kammern 104 und 106 ist eine Trennwand 116 angeordnet, die ebenfalls aus einem Hartkunststoff gebildet ist und so das Pipetierröhrchen 102 in dessen mittleren Bereich versteift.

Die Einsätze 110 und 114 können im Bereich ihrer jeweiligen Applizieröffnung bzw. -spitze mit einer Beflockung versehen sein, so dass die betreffende Substanz mittels der Beflockung auf die Applizierfläche aufgetragen werden kann.

Die Funktionsweise der Einsätze 110 und 114 in Verbindung mit den Führungsabschnitten 108 und 112 des Pipetierröhrchens 102 entspricht derjenigen des in den Eingriffsabschnitt eingreifendes Einsatzes der Ausführungsform nach den Figuren 1 bis 6, d. h. das Verlagern in Aktivierungsstellung bzw. in Sperrstellung erfolgt in entsprechender Weise durch Verdrehen des jeweiligen Einsatzes 110 bzw. 114 gegenüber dem Pipettierröhrchen 102.

## Patentansprüche

1. Vorrichtung zum Austragen einer fließfähigen Substanz, umfassend eine Außenhülse (14), die einen Behälter für die fließfähige Substanz bildet, sowie mindestens einen bereichsweise zylindrischen Einsatz (12), der einer Applikationsspitze (22) zugeordnet ist und der an einer Stirnseite offen ist und an der anderen Stirnseite geschlossen ist und an seinem Umfang mindestens eine Öffnung (34) hat, die mit der offenen Stirnseite verbunden ist, wobei die Außenhülse (14) gegenüber dem Einsatz (12) zwischen einer Sperrstellung, in der ein Fluidstrom zwischen der Außenhülse (14) und der Öffnung (34) des Einsatzes (12) mittels einer Dichteinrichtung (40) gesperrt ist, und einer Aktivierungsstellung verlagerbar ist, in welcher der Fluidstrom zwischen der Außenhülse (14) und der Öffnung (34) des Einsatzes (12) freigegeben ist, wobei der Einsatz (12) ein Außengewinde (20) hat, das in die Außenhülse (14) eingreift, wobei der fließfähige Stoff nach einem Verdrehen des Einsatzes (12) gegenüber der Außenhülse (14) und Freigabe der Öffnung (34) in den Einsatz (12) strömen kann, und wobei die Außenhülse zumindest bereichsweise aus einem verformbaren Kunststoff gebildet ist und der Einsatz (12) einen Ringbund aufweist, der ein Anschlag für den Einsatz (12) an der Außenhülse (14) und ein Betätigungsmittel zum Verlagern der Außenhülse (14) gegenüber dem Einsatz (12) ist, wobei die Außenhülse (14) als Zweikomponentenspritzgießteil ausgebildet ist und ein erster Abschnitt (46) der Außenhülse (14) zur Führung des Einsatzes (12) aus einem Hartkunststoff und ein zweiter Abschnitt der Außenhülse zum Austragen des fließfähigen Stoffes aus einem elastisch verformbaren Kunststoff gebildet ist.

2. Vorrichtung nach Anspruch 1, **dadurch gekennzeichnet, dass** das Außengewinde (20) des Einsatzes (12) in ein Innengewinde (18) der Außenhülse (14) eingreift.

3. Vorrichtung nach Anspruch 1, **dadurch gekennzeichnet, dass** das Außengewinde (20) des Einsatzes (12) ein selbstschneidendes Gewinde ist.

4. Vorrichtung nach einem der Ansprüche 1 bis 3, **gekennzeichnet durch** einen Anschlag, der die Aktivierungsstellung definiert.

5. Vorrichtung nach einem der Ansprüche 1 bis 4, **dadurch gekennzeichnet, dass** die Dichteinrichtung zwei Dichtlippen (38, 40) umfasst, die an der Außenhülse (14) innenseitig ausgebildet sind und deren Abstand größer ist als die maximalen Abmessungen der Öffnung (34) in axialer Richtung des Einsatzes (12).

6. Vorrichtung nach einem der Ansprüche 1 bis 5, **dadurch gekennzeichnet, dass** die Außenhülse ein Pipetierröhrchen ist.

7. Vorrichtung nach einem der Ansprüche 1 bis 6, **dadurch gekennzeichnet, dass** die Applikationsspitze des Einsatzes (12) mit einer Beflockung versehen ist.

8. Vorrichtung nach einem der Ansprüche 1 bis 7, **dadurch gekennzeichnet, dass** der Behälter zwei Kammern umfasst, die in dessen axialer Richtung hintereinander angeordnet sind und durch eine Trennwand voneinander getrennt sind und dass beide Kammern jeweils mit einem von zwei bereichsweise zylindrischen Einsätzen zusammenwirken, die jeweils einer Applikationsspitze zugeordnet sind.

## Claims

1. Device for dispensing a free-flowing substance, comprising an outer shell (14) forming a receptacle for the free-flowing substance, as well as at least one partially cylindrical insert (12) which is assigned to an application tip (22) and open at one end face and closed at the other end face and on its circumference features at least one orifice (34) being connected to the open end face, wherein the outer shell (14) can be shifted in relation to the insert (12) between a blocked position in which a fluid flow between the outer shell (14) and the orifice (34) of the insert (12) is blocked by means of a sealing device (40) and an activated position in which the fluid flow between the outer shell (14) and the orifice (34) of the insert (12) is released, wherein the insert (12) has a male screw thread (20) engaging with the outer shell (14), wherein the free-flowing substance can flow into the insert (12) after twisting of the insert (12) in relation to the outer shell (14) and release of the orifice (34), and wherein the outer shell is at least partially made of a deformable plastic material and the insert (12) comprises an annular collar acting as a stop for the insert (12) at the outer shell (14) and as an actuator for shifting the outer shell (14) in relation to the insert (12), wherein the outer shell (14) is designed as a two-component injection-molded part, and a first section (46) of the outer shell (14) being designed to guide the insert (12) is made of a rigid plastic material and a second section of the outer shell being designed to dispense the free-flowing substance is made of an elastically deformable plastic material.

2. Device according to Claim 1,
**characterized in that**
the male screw thread (20) of the insert (12) mates with a female screw thread (18) of the outer shell (14).

3. Device according to Claim 1,
**characterized in that**
the male screw thread (20) of the insert (12) is a self-cutting screw thread.

4. Device according to any of Claims 1 to 3,
**characterized by**
a stop defining the activated position.

5. Device according to any of Claims 1 to 4,
**characterized in that**
the sealing device comprises two sealing lips (38, 40) being formed on the inside of the outer shell (14) and having a spacing which is greater than the maximum dimensions of the orifice (34) in the axial direction of the insert (12).

6. Device according to any of Claims 1 to 5,
**characterized in that**
the outer shell is a pipetting tube.

7. Device according to any of Claims 1 to 6,
**characterized in that**
the application tip of the insert (12) is provided with a flock coating.

8. Device according to any of Claims 1 to 7,
**characterized in that**
the receptacle comprises two chambers which are consecutively disposed in the axial direction of the receptacle and which are separated from each other by a partition wall, wherein both chambers respectively interact with one of the two partially cylindrical inserts which are each assigned to an application tip.

## Revendications

1. Dispositif destiné à la distribution d'une substance coulante, comprenant une coque extérieure (14) formant un récipient pour la substance coulante, ainsi qu'au moins un insert (12) de forme partiellement cylindrique étant associé à une pointe d'application (22) et étant ouvert sur un côté frontal et fermé sur l'autre côté frontal et sur sa circonférence présentant au moins un orifice (34) étant relié avec le côté frontal ouvert, la coque extérieure (14) pouvant être déplacée par rapport à l'insert (12) entre une position bloquée dans laquelle un écoulement fluidique entre la coque extérieure (14) et l'orifice (34) de l'insert (12) est bloqué au moyen d'un dispositif de scellement (40) et une position activée dans laquelle l'écoulement fluidique entre la coque extérieure (14) et l'orifice (34) de l'insert (12) est libéré, l'insert (12) présentant un filet de vis mâle (20) venant s'engrener dans la coque extérieure (14), la substance coulante pouvant s'écouler dans l'insert (12) après retordage de l'insert (12) par rapport à la coque extérieure (14) et après libération de l'orifice (34), et dans lequel la coque extérieure est fabriquée au moins partiellement d'un matériau plastiquement déformable et l'insert (12) comprend un collet annulaire agissant comme une butée pour l'insert (12) sur la coque extérieure (14) et comme un actuateur pour le déplacement de la coque extérieure (14) par rapport à l'insert (12), la coque extérieure (14) étant façonnée en tant qu'une pièce moulée par injection à deux composants, et une première section (46) de la coque extérieure (14) étant conçue pour le guidage de l'insert (12) est fabriquée d'un matériau plastique rigide et une seconde section de la coque extérieure étant conçue pour la distribution de la substance coulante est fabriquée d'un matériau élastiquement déformable.

2. Dispositif selon la revendication 1,
**caractérisé en ce que**
le filet de vis mâle (20) de l'insert (12) vient s'accoupler avec le filet de vis femelle (18) de la coque extérieure (14).

3. Dispositif selon la revendication 1,
**caractérisé en ce que**
le filet de vis mâle (20) de l'insert (12) est un filet de vis auto-taraudant.

4. Dispositif selon l'une quelconque des revendications 1 à 3,
**caractérisé par**
une butée définissant la position activée.

5. Dispositif selon l'une quelconque des revendications 1 à 4,
**caractérisé en ce que**
le dispositif de scellement comprend deux lèvres de scellement (38, 40) étant formées sur le côté intérieur de la coque extérieure (14) et présentant un espacement qui est plus grand que les dimensions maximales de l'orifice (34) dans la direction axiale de l'insert (12).

6. Dispositif selon l'une quelconque des revendications 1 à 5,
**caractérisé en ce que**
la coque extérieure est un tube de pipetage.

7. Dispositif selon l'une quelconque des revendications 1 à 6,
**caractérisé en ce que**
la pointe d'application de l'insert (12) est munie d'un revêtement de flocage.

8. Dispositif selon l'une quelconque des revendications 1 à 7,
**caractérisé en ce que**
le récipient comprend deux chambres étant agencées de manière consécutive dans la direction axiale du récipient et étant séparées l'une de l'autre au moyen d'une paroi de partition, chacune des chambres respectivement interagissant avec un des deux inserts de forme partiellement cylindrique qui sont chacun associé à une pointe d'application.
